Europäisches Patentamt

European Patent Office

Office européen des brevets

(19)

(11) Publication number: **0 009 980**
**B1**

(12) # EUROPEAN PATENT SPECIFICATION

(45) Date of publication of patent specification: **31.08.83**

(51) Int. Cl.³: **A 61 B 10/00, G 01 N 1/10**

(21) Application number: **79302118.9**

(22) Date of filing: **04.10.79**

(54) **Urine specimen collecting device.**

(30) Priority: **10.10.78 US 949652**
**19.04.79 US 31427**
**24.07.79 US 60217**

(43) Date of publication of application:
**16.04.80 Bulletin 80/8**

(45) Publication of the grant of the patent:
**31.08.83 Bulletin 83/35**

(84) Designated Contracting States:
**AT BE CH DE FR GB IT LU NL SE**

(56) References cited:
**US - A - 3 499 327**
**US - A - 3 635 091**
**US - A - 3 750 647**
**US - A - 3 894 845**
**US - A - 3 943 770**
**US - A - 4 040 791**
**US - A - 4 064 760**
**US - A - 4 131 016**

(73) Proprietor: **SHS Enterprises, Ltd.**
**103 Edgewater**
**Newport Beach, California 92661 (US)**

(72) Inventor: **Kuntz, David H.**
**11810 Bel Terrace**
**Los Angeles California 90049 (US)**
Inventor: **Ingram, James Anthony**
**2528 Littleton Place**
**Costa Mesa California 92626 (US)**

(74) Representative: **Abnett, Richard Charles et al,**
**REDDIE & GROSE 16 Theobalds Road**
**London WC1X 8PL (GB)**

Courier Press, Leamington Spa, England

## Urine specimen collecting device

This invention relates to devices for collecting urine specimens for analysis in the detection and diagnosis of infection and disease.

Urine analysis is such a simple, common and useful diagnostic aid that the taking of a urine speciment is generally included in even the simplest of physical examinations. It is important not only as an indicator or detector of certain physical conditions and diseases, but it is also particularly useful in connection with the treatment of infections related to the urinary tract, which are much more common in females then males. However, the problem of collecting a suitable specimen is considerably more complex when a female patient is involved.

Thus, in the collecting of a urine specimen from a female patient, it is important to take the specimen as a "mid-stream" sample. When a female patient voids in the normal manner, the initial portion is more likely to contain contamination from foreign matter than that portion which follows. This is because the initial portion is expelled with less force and usually is voided as a mere trickle which is likely to trail along the labia and pick up whatever contamination is present in those areas. The mid-stream portion is expelled with the greatest force and is most likely to leave the urethral meatus directly as a stream without contacting the labia or, if it does, the labia will have had most of the contamination washed off by the initial portion.

Some practitioners go to considerable lengths in an effort to obtain an uncontaminated urine sample from a female patient. With the patient in a reclining position, an attendant cleanses the area adjacent the urethral meatus with a suitable solution in an effort to wash off the contamination which is generally present in that area. Even with such precautions, however, contamination may develop in the collected urine sample to a level sufficient to give a false or misleading indication of the bacteria level in the sample. Moreover, taking a sample under such conditions is an extremely awkward and uncomfortable experience for the patient and is fairly costly, since the patient is required to be present in the physician's office or a hospital with a special attendant, usually a nurse, administering the procedure.

What has been needed is some simple but effective, fool-proof device, preferably disposable after a single use, which a patient can use with a minimum of instructions and without assistance, while voiding in the normal manner. The device should have the capability of receiving the entire amount of voided urine, rejecting the initial portion, selecting a portion corresponding to the mid-stream sample and transferring it to a specimen container, and rejecting the remainder of the voided urine by directing it, together with any overflow from the specimen container, into the toilet on which the patient is positioned in the normal attitude.

U.S. Patent Specification No. 4,040,791 describes a device for selectively collecting a mid-stream portion of voided urine comprising a collector with a receiving chamber and a drain exit for the first voided portion of urine, and a transfer exit for the mid-stream portion leading to a detachable specimen container, the device further having an overflow passage for urine in excess of the specimen to be retained in the container.

The present invention is characterized in that a well depends centrally from the bottom of the receiving chamber, the drain exit being at the bottom of the well, the specimen container is annular and surrounds the well, and the transfer exit is formed by openings in the peripheral wall of the collector.

In one arrangement in accordance with the invention, the collector (apart from the specimen container) is much like a funnel in configuration with a handle extending from the edge thereof. The cone-shaped portion of the funnel corresponds to the collection chamber; the lower central tube of the funnel constitutes the bleed-off conduit and is closed off at the lower end thereof except for an aperture of limited size to control the bleed-off of the first-voided urine at a limited rate. Near the juncture of the collection chamber and the bleed-off conduit is a plurality of apertures or windows, equally spaced about the periphery of the bleed-off conduit, constituting the second exit for communicating with and transferring urine to a specimen container when the specimen container is in place. The specimen container is toroidal in planar cross-section, has both inner and outer walls, and (when mounted together with the collector) surrounds the bleed-off conduit. The specimen container is provided with a flexible lid having a central opening. Mating lip and recess surfaces around the periphery of the specimen container and lid, respectively, provide an effective circumferential seal between lid and container. The portion of the lid surrounding the central opening is spaced upwardly from the upper end of the inner wall of the container, thus defining a circumferential opening which is adjacent the windows at the base of the cone-shaped portion of the collector so as to admit urine therethrough. A mating ring and recess serve to retain the specimen container on the blood-off conduit, while permitting the collector and container to be separated easily, when desired. Preferably a plurality of upwardly extending shields or dams are located about the base of the collecting chamber in registration with the second exit apertures so as to shield those apertures from urine being voided into the collector until after the first-voided portion fills the bleed-off conduit.

An overflow conduit connecting with the chamber at an overflow exit positioned significantly above the second exit is provided to remove any excess urine from the collector and prevent it from running over its uppermost rim. This overflow exit is also provided with a shield to block urine as voided from passing through the overflow exit.

In a first alternative arrangement of the embodiment described, the overflow exit and overflow conduit connect to the side of the collection chamber in a position radially displaced from the central axis of the device.

In a second alternative, arrangement, the overflow conduit is centrally positioned as a standpipe extending upwardly from the closed off exit of the bleed-off conduit. In the latter case, the bleed-off exit opening may be radially displaced and positioned between the wall of the overflow conduit and the inner wall of the bleed-off conduit.

In still another arrangement, the collector has one or more of the exit windows blocked off and is molded with one or more aligned recessed troughs extending downwardly along the bleed-off conduit. This serves in conjunction with the adjacent opening in the speciment container as an overflow passage. Also a flow director or deflector is mounted interiorly of the collector in the region of the exit windows to direct urine initially into the bleed-off conduit from whence it flows into the specimen container. When the specimen container is filled to overflowing, urine flows out of the specimen container at the overflow passage and down the recessed trough(s) and into the toilet. Any further voided urine follows this overflow path out of the device.

In accordance with an aspect of the invention, the flexible lid of the specimen container and the upper edge of the inner wall of the container are configured to mate in sealing relationship when the central portion of the lid is pushed downwardly to engage the inner wall of the container. This serves to complete the closure of the specimen container after the selected urine sample has been collected therein.

To facilitate the sealing operation, the height of the specimen container is made to exceed the length of the bleed-off conduit by approximately 6.3 mm to 12.7 mm (1/4 to 1/2 inch). The collector is provided with a circumferential shoulder surrounding the bleed-off conduit near the juncture of the bleed-off conduit and the collection chamber. This shoulder is provided with a circumferential recess capable of receiving the upper edge of the inner wall of the specimen container when the two are pushed together. When this is done, the central ring portion of the flexible lid is pushed downwardly to engage a circumferntial lip surrounding the inner wall of the container, thus retaining the central portion of the lid in the downward position and effectively closing off and sealing the circumferential opening which previously existed between the lid and the upper edge of the inner wall of the container.

By virtue of this arrangement, it is intended that after the patient has used the collector device and left it for the nurse or other medical assistant, the nurse simply pushes downward on the upper circumferential edge of the cone-shaped collecting chamber, thereby driving the lid of the specimen container into sealing relationship with the inner wall of the container. This not only serves to seal the previously existing opening between the lid and the inner wall, but it reinforces the outer circumferential seal between the lid and the outer wall of the container by developing a downward bias on the lid. Thereafter, the nurse simply grasps the specimen container and lid with one hand and removes the collector portion by pulling it upward and away from the specimen container portion. Later, when it is desired to open the specimen container, the lid is simply unsealed by lifting upward relative to the specimen container at the circumferential edge thereof. This serves to release both the inner and outer seals so that the lid can be removed from the container. These various features and aspects of the present invention result in an improved urine specimen collecting device which is extremely effective in collecting the desired contaminant-free mid-stream portion during use, is readily sealable and separable for retention of the specimen container while the collector proper can be thrown away, is simple, lightweight, cheap to manufacture and easy to use without elaborate instruction.

In the accompanying drawings:

Fig. 1 is an elevational view of one particular device in accordance with the invention;

Fig. 2 is an exploded view of the device shown in Fig. 1;

Fig. 3 is a plan view of the device of Fig. 1;

Fig. 4 is a quarter-sectional view of a portion of the device depicted in Fig. 2, taken along the line 4—4 thereof;

Fig. 5 is a quarter-sectional view of a portion of the device of Fig. 1, taken along the line 5—5 of Fig. 3;

Fig. 6 is a sectional view similar to Fig. 5, showing the assembly of the various elements making up the device of the invention;

Fig. 7 is a plan view of an alternative embodiment of the invention;

Fig. 8 is an elevational view, partially broken away, of a portion of the alternative embodiment of Fig. 7;

Fig. 9 is an exploded elevational view of still another device in accordance with the invention;

Fig. 10 is a sectional view of a portion of the device depicted in Fig. 9 taken along the plane of the drawing and looking upward from the drawing plane;

Fig. 11 is a bottom view of the portion of the collector element shown in Fig. 10;

Fig. 12 is an upper plan view of the portion of the collector shown in Fig. 10;

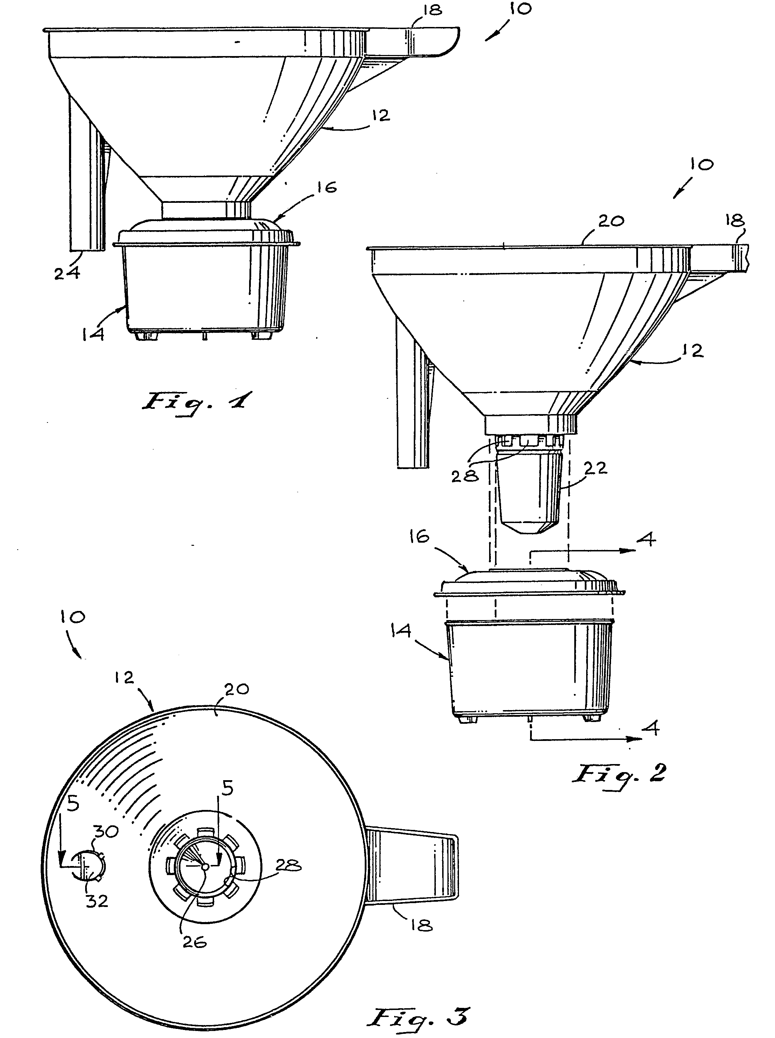

One preferred embodiment of the present invention is depicted in Figs. 1—3 of the drawings. As is shown particularly in the exploded view of Fig. 2, the collecting device 10 comprises a collector 12, specimen container 14 and specimen container lid 16.

The collector 12 is a generally funnel-shaped element having a handle 18, a cone-shaped receiving chamber 20, a bleed-off conduit 22 at the lower end of the cone-shaped receiving chamber 20, and an overflow conduit 24 which extends through the sidewall of the receiving chamber 20 and is attached thereto.

The collector 12 is provided with three exits for the release of urine therefrom. The first exit is a bleed-off exit 26 comprising a small hole at the bottom of the bleed-off conduit 22, the bottom of which is otherwise closed off and thereby constitutes a pocket or recess for receiving the first-voided urine from the chamber 20. The second exit comprises a plurality of apertures or windows 28 near the bottom of the cone-shaped portion of the chamber 20. As will be shown in detail hereinbelow, these windows 28 communicate with the interior of the specimen container 14 when the constituent elements are assembled in the configuration shown in Fig. 1. A third exit 30 is located in the side of the chamber 20 above the second exit 28 and communicates with the interior of the overflow conduit 24. The overflow exit 30 is provided with a shield 32 to block the overflow of urine via that exit 30 until the urine level in the collector 12 rises to the overflow exit 30.

As seen in Fig. 4, which is a quarter-sectional view of a portion of the device of Fig. 2, taken along the line 4—4 and looking in the direction of the arrows, the specimen container 14 is somewhat toroidal in form, having an upstanding hollow center section surrounded by an inner cylindrical wall 42 and an outer cylindrical wall 44. The storage space for urine collected in the container 14 is between the inner and outer walls 42, 44. This space 46 is open at the top of the container 14. Legs 47 are mounted at the base of the container 14.

Near the top of the outer cylindrical wall 44 is an outwardly protruding lip or rim 48 which extends circumferentially about the specimen container 14. A similar protruding lip or rim 50 projects radially inwardly from the inner wall 42 and extends circumferentially about the hollow space defined by the wall 42. Near the top of the wall 42 in the side facing the space 46 is a recess or slot 52 which extends circumferentially about the specimen container inner wall 42.

The lid 16 is provided with an inner circumferential recess 54 which serves to mate with the rim 48 of the container 14 when the lid 16 is mounted on the container 14, thus retaining the lid thereon in sealing relationship about the outer edge of the container 14. The lid 16 is provided with a plurality of downwardly depending, projecting stop pins 55 which, when the lid 16 is first placed on the container 14, bear against a ledge 56 on the inner wall 42 of the container 14 and maintain a space between the ring 58 about the central opening of the lid 16 and the upper edge 59 of the inner wall 42 of the container 14. These stop pins 55 are flexible, as is the lid 16, so that upon the application of downward force sufficient to bend the pins 55, they give away and permit an inwardly projecting rim 60 of the lid 16 to move downwardly into engagement with the circumferential recess 52, thus establishing a seal between the inner rim 60 of the lid 16 and the upper end of the inner wall 42 of the container 14. When mounted in such fashion, the storage space 46 for the urine which is collected in the container 14 is completely sealed — at the outer edge by the seal between the outer rim 48 and the recess 54, and at the inner edge by the seal between the inner rim 60 and the recess 52.

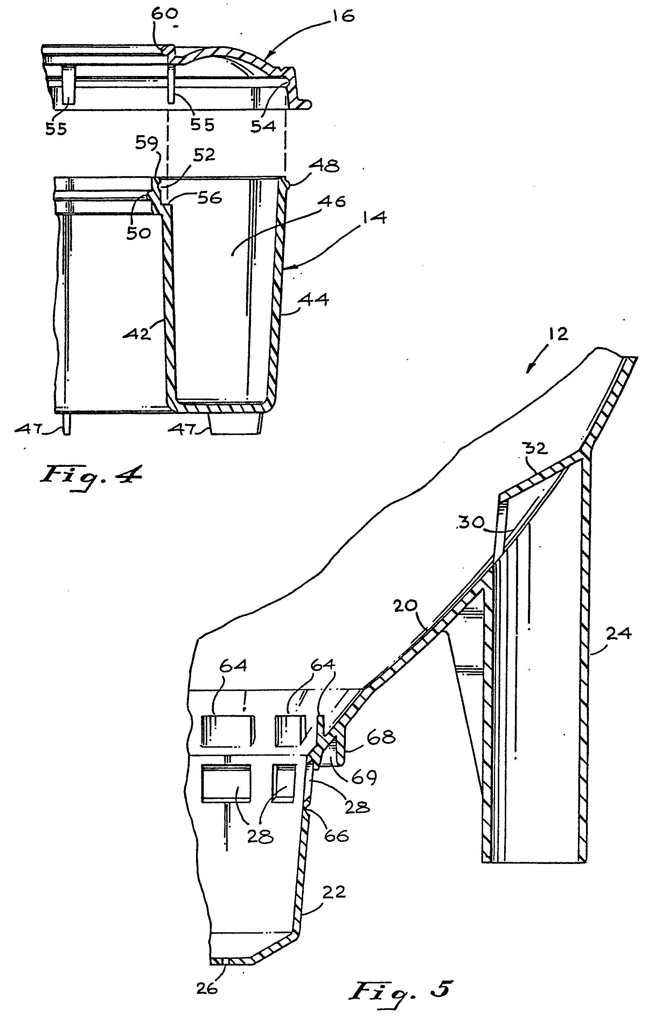

Referring now to Fig. 5, a sectional view taken along the line 5—5 of Fig. 3 and looking in the direction of the arrows, certain structural details of the collecting device of our invention are shown therein. Fig. 5 shows the sidewall of the receiving chamber 20 in the region of the overflow conduit 24 and the collecting exit comprising the windows or apertures 28. The overflow exit 30 may be seen extending through the sidewall of the receiving chamber 20 and communicating with the overflow conduit 24. As shown, the exit 30 is protected by the shield 32 to minimize the splashing of urine through the exit 30. Shields or barriers 64 serving as tiny dams are mounted respectively above the windows 28 to interfere with the flow of the first-voided urine through the windows 28. These barriers 64 channel the urine through the passages between adjacent barriers 64, thus diverting the urine from the windows 28 until the urine rises in the bleed-off conduit 22 to the level of the windows 28.

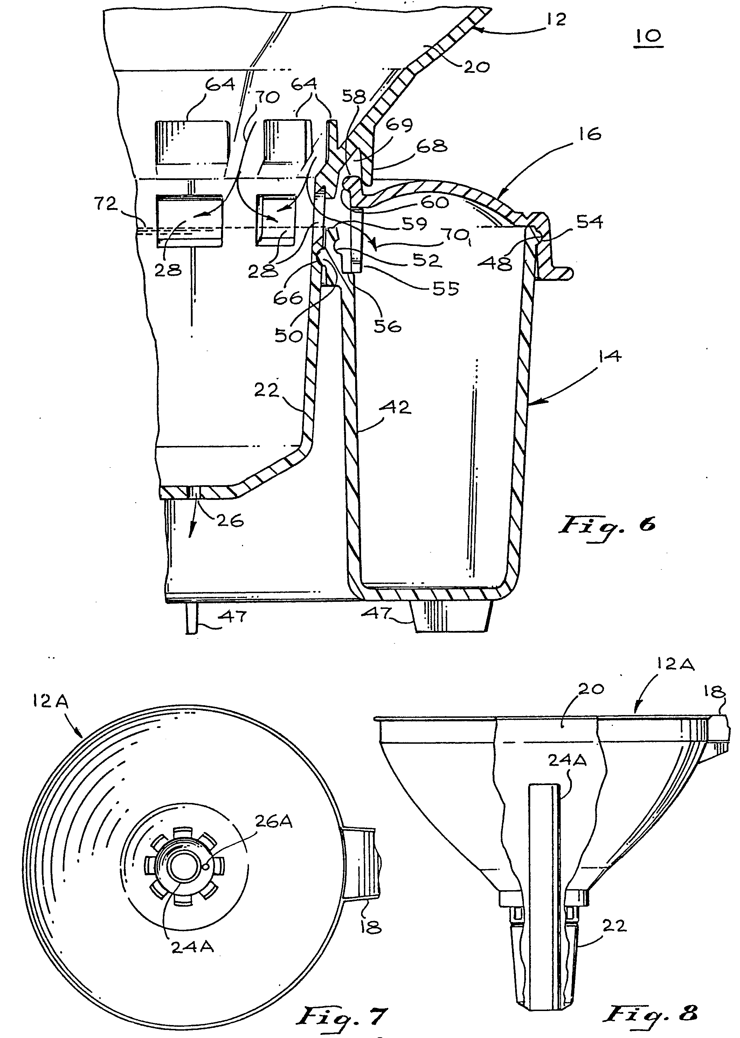

The sidewall of the bleed-off conduit 22 is provided with a circumferential recess 66 positioned just below the windows 28. This serves to receive the inner rim 50 of the container 14, thus retaining the container 14 in sealing relationship to the collector 12 when the two are affixed together. Of course, the lid 16 will be positioned between the collector 12 and container 14 when mounted in this fashion (see Fig. 6).

The collector 12 is provided with a downwardly depending, circumferential skirt or lip 68 which defines, with the wall of the bleed-off conduit 22, a circumferential recess 69.

Fig. 6 is a sectional view similar to that of Fig. 5 but showing the container 14, lid 16 and collector 12 assembled together as prepared for shipment and for operative use in selectively collecting a mid-stream urine specimen. The lid 16 is shown in place on the container 14 with the outer periphery of the lid sealed to the container 14 by virtue of the mating of the rim 48 in the circumferential recess 54. Similarly, the inner wall of the specimen container 14 is sealably engaged to the bleed-off conduit 22, and thereby retained thereon, by the mating relationship of the inner rim 50 in the circumferential recess 66. The central circumferential ring 58 of the lid 16 surrounding the central opening thereof is partially within the recess 69 and bearing against the skirt 68. A stop in 55 is shown bearing against the ledge 56, thus maintaining the space between the upper edge 59 of the wall 42 and the lid 16. This space is adjacent the windows 28 and extends circumferentially about the assembly, interrupted only by the spaced-apart pins 55.

When the collector device is used by a patient, urine is voided into the receiving chamber 20 of the collector 12. The first-voided urine flows down into the bleed-off conduit 22 and begins bleeding out the bleed-off exit opening 26; if the urine initially drops onto the wall of the receiving chamber 20, it flows down the wall and in the channels between the barriers or dams 64, as indicated by the arrows 70. When the urine level rises to the windows 28, as indicated by the line 72, urine then begins flowing through the windows 28 and through the entrance space between the lid 16 and the inner wall 42 of the specimen container 14, as further indicated by the arrows 70. As voiding continues, the mid-stream portion of the voiding is directed in the manner described into the specimen container 14. As the specimen container 14 fills with urine, the air which was initially in the empty container 14 is permitted to escape out the same entrance space and the windows 28. further voiding after the specimen container 14 is full backs up into the receiving chamber 20 until the level of the overflow exit 30 (Fig. 5) is reached, after which the overflow urine runs out the overflow conduit 24. When voiding is terminated, the urine remaining in the collector bleeds off through the bleed-off conduit 22 and exit 26, until the collector 12 is emptied.

At this point, the patient will normally deliver the collector and specimen container with the specimen contained therein to the nurse or other medical assistant who thereafter places the device 10 on a counter, table or other horizontal surface and bears down on the upper edge of the collector 12. This causes skirt 68 to bear down against the adjacent surface of the lid 16 as the ring 58 of the lid moves further into the recess 69 and causes the semi-flexible pins 55 to bend and slip off the ledge 56. Further downward movement of the collector 12 relative to the specimen container 14 causes the inner rim 60 of the lid 16 to move downwardly into engagement with the circumferential recess 52, effecting the seal between the rim 60 and the recess 52 and thus preventing any escape of the specimen collected within the container 14. The collector 12 is then pulled upwardly, relative to the lid 16 and container 14, releasing the retention of the container 14 on the bleed-off conduit 22 at the recess 66 and separating the collector from the specimen container. The collector 12 is then discarded and the specimen container retained for processing of the collected specimen.

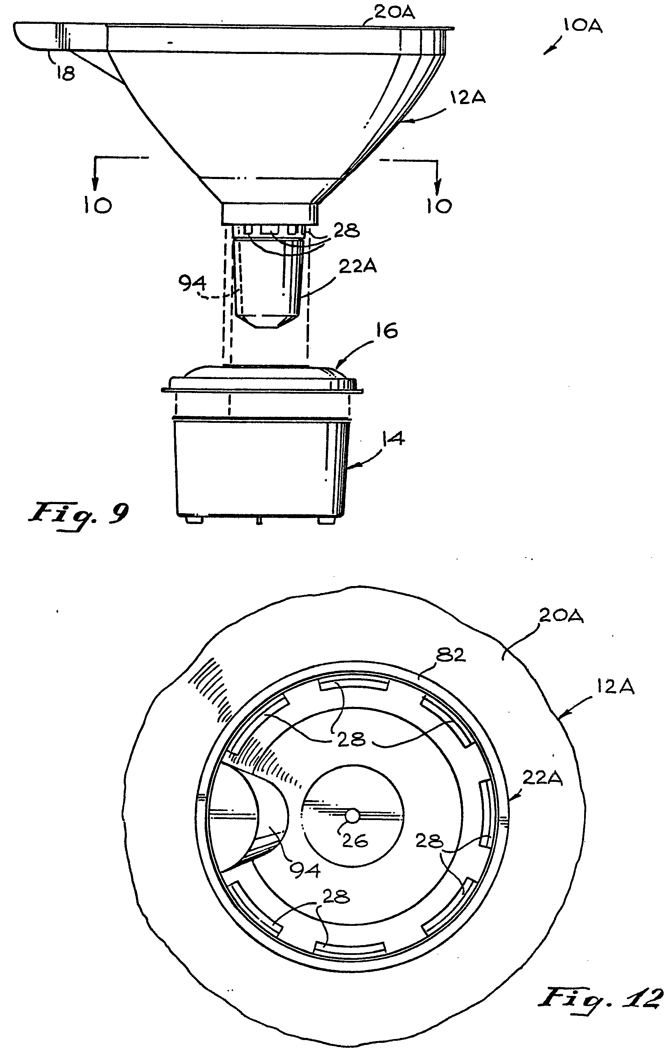

An alternative embodiment of the invention is shown in Figs. 7 and 8. In this embodiment, the elements of the collecting device 10 are the same as shown in Figs. 1—6, except that the overflow conduit 24A is centrally located within the bleed-off conduit 22. The bleed-off exit opening 26A is shown at the bottom of the bleed-off conduit 22 (see Fig. 7) but located between the wall of the overflow conduit 24A and the inner wall of the bleed-off conduit 22. The bottom of the overflow conduit 24A is of course open to permit the escape of urine overflowing through the conduit 24A. Utilization of this alternative embodiment of the collector 12A, when assembled in combination with the specimen container 14 and the lid 16, is the same as has been described for the use of the embodiments of Figs. 1—6.

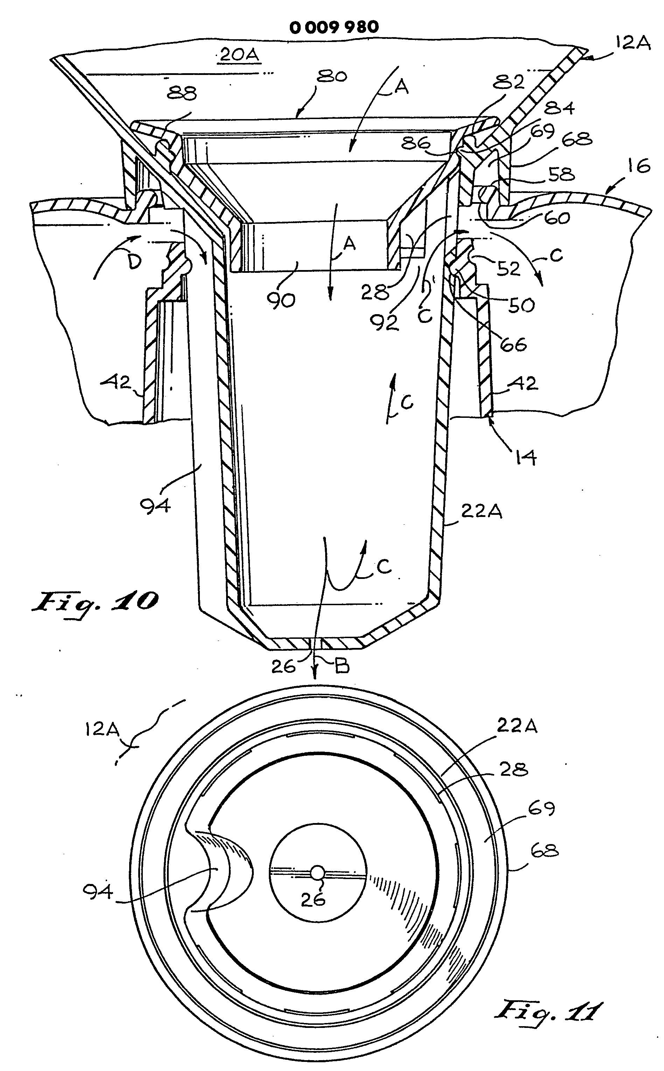

The device depicted in Figs. 9—12 is a variant of the device shown in Figs. 1—6. Where like elements are included, the same reference numerals have been employed. As shown particularly in Figs. 9 and 10, the device 10A includes a collector 12A and an integral specimen container 14 and associated lid 16. The notable difference is the lack of an overflow conduit per se such as the conduit 24 shown in Fig. 1.

The collector 12A has a receiving chamber 20 in the upper portion of the generally funnel-shaped configuration opening into a downwardly depending bleed-off conduit 22A, near the bottom of which is a first or drain exit 26 for draining urine from the collector. A second or transfer exit is provided by the window openings 28 which are spaced about the periphery of the upper portion of the exit or bleed-off conduit 22A except on the side in line with the handle 18. At this point, the periphery of the collector 12A is closed and, further, the collector is molded with a recessed trough 94 (see Fig. 10) extending downwardly along the bleed-off conduit 22A. This trough 94 serves to provide an overflow passage for any urine tending to overflow the specimen container 14, thus permitting such excess urine to flow downwardly and outwardly from the urine collecting device 10A and directly into the toilet.

To provide more effective control of the flow of urine being voided into the collecting device 10A, a diverter or deflector insert 80 may be mounted in the device at the bottom of the receiving chamber 20. This deflector 80 is configured to adapt to the interior shape of the collector 12A, i.e. the diverter 80 is also generally funnel-shaped, and is provided with an outer lip 88 to collect urine flowing down the walls of the receiving chamber 20 and direct it into the bleed-off conduit 22A through the lower opening 90 of the diverter 80. The interior of the collector 12A is provided with a circumferential wall 82 projecting upwardly by a sufficient distance to engage and retain the diverter 80. This wall is provided with an inwardly projecting ring 84 adapted to mate with a corresponding circumferential recess 86 in the diverter 80. Thus, the diverter 80 is retained in the position shown in Fig. 10 after it is simply snapped in position by downward pressure on the diverter 80 during assembly. The portion of the collector 12A shown in Fig. 12 is as it appears prior to insertion of the diverter 80. The diverter 80, in use, serves to prevent urine from flowing out the windows 28 and into the speciment container 14 until after the urine has entered the bleed-off conduit 22A, thus insuring that none of the unwanted, first-voided urine can inadvertently enter the specimen container.

In use, the collecting device 10A is held by the female subject, grasped by the handle 18 between thumb and forefinger and held in a proper position for receiving voided urine while the subject is sitting on a toilet. Use in the normal attitude thus orients the device at an angle which locates that portion of the opening between the portion 50 of the specimen container 14 and the inner ring portion 58 of the lid 16 which is adjacent the trough 94 somewhat higher than the remainder of the opening. This thus provides for the escape of air which might otherwise be entrapped within the specimen container 14 and prevent it from being adequately filled with the desired specimen. Furthermore, this portion, in conjunction with the recessed trough 94, establishes an overflow passage, permitting excess urine to flow out of the device, once the specimen container 14 is substantially filled. Thus, any excess urine is rapidly directed out of the device and into the toilet via this overflow passage including the trough 94 until voiding is terminated. At that point, the subject need only wait a very brief interval until the small amount of urine in the bleed-off conduit 22A drains out through the drain hole 26 before she can turn the collecting device 10A with specimen contained therein over to the nurse or other attendant.

As particularly shown in Fig. 10, urine received into the collector 12A flows downwardly in the direction of the arrows A into the bleed-off conduit 22A. Thereafter, the flow divides as shown by arrows B and C. A portion of the urine flows out through the drain 26 (arrow B) while the remaining portion is directed upwardly (arrows C) to fill the bleed-off conduit 22A and ultimately begin overflowing into the region 92 and entering the specimen container 14. When the level of the urine in the specimen container 14 reaches the overflow passage on the left-hand side of Fig. 10, it flows outwardly (arrows D) from the specimen container 14 and downwardly through the space between the bleed-off conduit 22A and the wall 42 of the specimen container 14 via the trough 94. When voiding is finished, any urine remaining in the collector 12A drains through the drain opening 26 until the collector per se is empty. The only urine remaining is in the specimen container 14. Thereafter, the collecting device 10A is processed as previously described to seal the lid 16 on the container 14 and remove the specimen and container from the collector, which is discarded.

## Claims

1. A device for selectively collecting a midstream portion of voided urine comprising a collector (12) with a receiving chamber (20) and a drain exit (26) for the first voided portion of urine, and a transfer exit (28) for the mid-stream portion leading to a detachable specimen container (14), the device further having an overflow passage (24) for urine in excess of the specimen to be retained in the container characterized in that a well (22) depends centrally from the bottom of the receiving chamber (20), the drain exit (26) being at the bottom of the well, the specimen container (14) is annular and surrounds the well, and the transfer exit (28) is formed by openings in the peripheral wall of the collector.

2. A device as claimed in claim 1 characterized in that the specimen container and the well of the collector are formed to snap into releasable engagement with one another (50, 66).

3. A device as claimed in claim 1 or 2 characterized in that the overflow passage (24) is formed between an overflow exit at the top of the specimen container and the outer surface (94) of the peripheral wall of the well.

4. A device as claimed in claim 1, 2 or 3 in which the container has a flexible lid (16) sealed thereto around its outer periphery and with a central opening which is spaced from the container.

5. A device as claimed in claim 4 in which the lid (16) is flexible and its inner periphery is engageable by snap action with the container such that after filling of the container the inner periphery of the lid can be forced downward to seal the container.

6. A device as claimed in claim 5 wherein the inner wall (42) of the container (14) is provided with a circumferential recess near the upper end of the wall for sealingly engaging a mating portion (60) of the container lid.

7. A device as claimed in claim 4, 5 or 6 in which the lid is a snap fit with the container around its outer periphery.

8. A device as claimed in claim 7 wherein the outer wall of the container (14) includes an outwardly extending circumferential rim (48) near the upper end of the outer wall for engaging a mating circumferential recess (54) in the inner surface of the lid about the periphery thereof.

9. A device as claimed in any of claims 4 to 8, wherein the collector further comprises a circumferentially extending, downwardly directed skirt (68) configured to bear against the central portion of the lid upon the application of force upon the collector in the direction toward the container to deflect the inner portion of the lid into sealing engagement with the inner wall of the container.

10. A device as claimed in any of claims 1 to 9 in which the transfer exit comprises a plurality of circumferentially spaced windows (28) in the vicinity of the junction between the receiving chamber and the well.

11. A device as claimed in any of the preceding claims in which the receiving chamber is cone-shaped and leads into a tubular well (22).

12. A device as claimed in any of the preceding claims including means for diverting urine from the transfer exit until the well is filled.

13. A device as claimed in claim 12 in which the diverting means comprise a generally funnel-shaped diverter (80) inserted in the lower portion of the receiving chamber and extending downwardly to overlap the transfer exit.

## Revendications

1. Dispositif destiné à collecter sélectivement une partie centrale d'urine émise, ce dispositif comprenant un collecteur (12) comportant une chambre (20) de réception et une sortie (26) de vidange de la partie d'urine émise en premier, et une sortie (28) de transfert de la partie centrale de l'urine, conduisant à un conteneur (14) d'échantillon détachable, le dispositif comportant en outre un passage ou conduit (24) de débordement de l'urine en excès par rapport à l'échantillon à retenir dans le conteneur, dispositif caractérisé en ce qu'un puits (22) fait saillie, en position centrale, du bas de la chambre (20) de réception vers le bas, la sortie (26) d'égouttage étant au bas du puits, le conteneur (14) d'échantillon est annulaire et entoure le puits, et la sortie (28) de transfert est formée par des ouvertures ménagées dans la paroi périphérique du collecteur.

2. Dispositif selon la revendication 1, caractérisé en ce que le conteneur de l'échantillon et le puits du collecteur sont formés de manière à s'enclencher en un contact libérable l'un avec l'autre (50, 66).

3. Dispositif selon la revendication 1 ou 2, caractérisé en ce que le passage ou conduit (24) de débordement est formé entre une sortie de débordement au sommet du conteneur d'échantillon et la surface externe (94) de la paroi périphérique du puits.

4. Dispositif selon la revendication 1, 2 ou 3, dans lequel le conteneur comporte un couvercle (16) souple qui lui est relié de manière étanche autour de sa périphérie externe, ainsi au'une ouverture centrale espacée du conteneur.

5. Dispositif selon la revendication 4, dans lequel le couvercle (16) est souple et sa périphérie interne peut venir par enclenchement au contact du conteneur de manière qu'après emplissage du conteneur, la périphérie interne du couvercle puisse être repoussée vers le bas de manière à assurer la fermeture étanche du conteneur.

6. Dispositif selon la revendication 5, dans lequel la paroi interne (42) du conteneur (14) comporte un creux circonférentiel près de l'extrémité supérieure de la paroi pour venir en contact étanche avec une partie (60) correspondante du couvercle de conteneur.

7. Dispositif selon la revendication 4, 5 ou 6, dans lequel le couvercle s'adapte par enclenchement sur le conteneur autour de sa périphérie externe.

8. Dispositif selon la revendication 7, dans lequel la paroi externe du conteneur (14) comprend un rebord circonférentiel (48) faisant saillie vers l'extérieur près de l'extrémité supérieure de la paroi externe pour venir au contact d'un creux circonférentiel (54) correspondant dans la surface interne du couvercle autour de la périphérie de celui-ci.

9. Dispositif selon l'une quelconque des revendications 4 à 8, dans lequel le collecteur comprend en outre une jupe (68) dirigée vers le bas, s'étendant circonférentiellement, configurée de manière à porter contre la partie centrale du couvercle lors de l'application d'une force sur le collecteur en direction du conteneur pour infléchir la partie interne du couvercle et la faire venir en contact étanche avec la paroi interne du conteneur.

10. Dispositif selon l'une quelconque des revendications 1 à 9, dans lequel la sortie de transfert comprend plusieurs fenêtres (28) espacées circonférentiellement au voisinage de la jonction entre la chambre de réception et le puits.

11. Dispositif selon l'une quelconque des revendications précédentes, dans lequel la chambre de réception est de forme conique et débouche dans un puits tubulaire (22).

12. Dispositif selon l'une quelconque des revendications précédentes, comprenant un moyen pour dévier l'urine de la sortie de transfert jusqu'à emplissage du puits.

13. Dispositif selon la revendication 12, dans lequel le moyen de déviation comprend un élément (80) de déviation, à forme générale d'entonnoir, inséré dans la partie inférieure de la chambre de réception et s'étendant vers le bas pour recouvrir la sortie de transfert.

## Patentansprüche

1. Vorrichtung zum getrennten Auffangen von nicht gleich zu Anfang ausgeschiedenem Urin, die einem Sammler (12) mit einem Auf-

fangbereich (20) und einem Abfluß (26) für den am Anfang ausgeschiedenen Urin und einem Fülleinlaß (28) für den nicht gleich zu Anfang ausgeschiedenen Urin, der zu einem Probenbehälter (14) geleitet wird, aufweist und weiterhin einen Überlaufkanal (24) für den Urin enthält, der über die im Behälter aufzunehmende Menge hinausgeht, dadurch gekennzeichnet, daß ein Behälter (22) mittig mit dem unteren Teil des Auffangbereichs (20) verbunden ist, der Abfluß (26) sich am Boden des Behälters befindet, der Probenbehälter (14) ringförmig ist und den Behälter umgibt und daß der Fülleinlaß (28) durch Öffnungen im Rand des Sammlers gebildet wird.

2. Vorrichtung nach Anspruch 1, dadurch gekennzeichnet, daß der Probenbehälter und der Behälter des Sammlers so ausgebildet sind, daß sie miteinander in lösbarer Verbindung einrasten können (50, 66).

3. Vorrichtung nach einem der Ansprüche 1 oder 2, dadurch gekennzeichnet, daß der Überlaufkanal (24) zwischen dem Überlaufaustritt am oberen Rand des Probenbehälters und der Außenfläche (94) der Umfangswandung des Behälters gebildet wird.

4. Vorrichtung nach einem der Ansprüche 1, 2 oder 3, dadurch gekennzeichnet, daß der Probenbehälter einen flexiblen Deckel (16) besitzt, der mit ihm entlang seines äußeren Ümfangs luftdicht verschlossen un mit einer zentralen Öffnung versehen ist, die einen Abstand zum Probenbehälter aufweist.

5. Vorrichtung nach Anspruch 4, dadurch gekennzeichnet, daß der Deckel (16) flexibel ist und sein innerer Umfang mittels Schnappverbindung mit dem Probenbehälter in Eingriff gebracht werden kann, indem er nach dem Füllen des Probenbehälters zu seinem luftdichten Verschließen niedergedrückt wird.

6. Vorrichtung nach Anspruch 5, dadurch gekennzeichnet, daß die Innenwand (42) des Probenbehälters (14) an ihrem oberen Ende eine um ihren Umfang verlaufende Vertiefung zum luftdichten Eingreifen eines dazu passenden Teils (60) des Probenbehälterdeckels aufweist.

7. Vorrichtung nach einem der Ansprüche 4, 5 oder 6, dadurch gekennzeichnet, daß der Deckel auf dem Probenbehälter entlang seinem äußeren Umfang durch eine Schnappverbindung befestigt ist.

8. Vorrichtung nach Anspruch 7, dadurch gekennzeichnet, daß die Außenwand des Probenbehälters (14) eine sich entlang ihres Umfangs nach außen erstreckende Wulst (48) nahe ihres oberen Endes aufweist, um sie mit einer sich um den Umfang der Innenfläche des Deckels erstreckenden dazu passenden Vertiefung (54) in Eingriff zu bringen.

9. Vorrichtung nach einem der Ansprüche 4—8, dadurch gekennzeichnet, daß der Sammler weiterhin einen sich um den Umfang erstreckenden, nach unten gerichteten Flansch (68) aufweist, der so ausgebildet ist, daß er bei Krafteinwirkung auf den Sammler in Richtung zum Probenbehälter gegen den mittleren Bereich des Deckels drückt, um den inneren Bereich des Deckels in luftdichten Eingriff mit der Innenwand des Probenbehälters zu bringen.

10. Vorrichtung nach einem der Ansprüche 1—9, dadurch gekennzeichnet, daß der Fülleinlaß mehrere Fenster (28) aufweist, die in gewissen Abständen in der Nähe der Verbindung zwischen dem Auffangbereich und dem Behälter entlang dessen Umfang angeordnet sind.

11. Vorrichtung nach einem de Ansprüche 1—10, dadurch gekennzeichnet, daß der Auffangbereich kegelförmig ist und in einen röhrenförmigen Behälter (22) führt.

12. Vorrichtung nach einem der Ansprüche 1—11, dadurch gekennzeichnet, daß Vorrichtungen vorhanden sind, um Urin vom Fülleinlaß abzuleiten, wenn der Behälter gefüllt ist.

13. Vorrichtung nach Anspruch 12, dadurch gekennzeichnet, daß die Ableitvorrichtung eine im allgemeinen trichterförmige Ableitung (80) enthält, die in den unteren Teil des Auffangsbereichs eingesetzt ist und sich nach unten erstreckt, um den Fülleinlaß zu überdecken.

**0 009 980**

Fig. 1

Fig. 2

Fig. 3

0 009 980

Fig. 4

Fig. 5

2

0 009 980

Fig. 6

Fig. 7

Fig. 8

0 009 980

Fig. 9

Fig. 12

4

**0 009 980**

Fig. 10

Fig. 11